# FASCICULE DE BREVET EUROPEEN

(11) **EP 2 694 462 B1**
(45) Date de publication et mention de la délivrance du brevet: **07.10.2015**
(21) Numéro de dépôt: 12718286.3
(22) Date de dépôt: 30.03.2012
(51) Int. Cl.: C07C 43/23, C07C 49/245, A61K 8/35, A61Q 5/00, A61K 8/34

(54) **UTILISATION COMME ANTIPELLICULAIRE DE COMPOSÉS (ÉTHOXYHYDROXYPHÉNYL)ALKYLCÉTONE OU ÉTHOXYHYDROXYALKYLPHÉNOL**
VERWENDUNG VON (ETHOXYHYDROXYPHENYL)ALKYLKETON- ODER ETHOXYHYDROXYALKYLPHENOLVERBINDUNGEN ALS ALS ANTISCHUPPENMITTEL
USE, AS ANTIDANDRUFF AGENT, OF (ETHOXYHYDROXYPHENYL)ALKYL KETONE OR ETHOXYHYDROXYALKYLPHENOL COMPOUNDS

(30) Priorité: 01.04.2011 FR 1152804; 07.04.2011 US 201161472665 P
(43) Date de publication de la demande: 12.02.2014
(73) Titulaire: L'Oréal, 75008 Paris (FR)
(72) Inventeur: DALKO, Maria, F-78000 Versailles (FR)
(74) Mandataire: Kromer, Christophe
(86) Numéro de dépôt international: PCT/FR2012/050696
(87) Numéro de publication internationale: WO 2012/131274

(56) Documents cités:
- WO-A1-2011/039445
- JP-A- 2007 238 569
- US-A1- 2004 171 698
- DATABASE CAPLUS [Online] CHEMICAL ABSTRACTS SERVICE, COLUMBUS, OHIO, US; YANG, ZHENG ET AL: "Shampoo containing extracts of Zingiber officinale with dandruff eliminating function", XP002658770, extrait de STN Database accession no. 2006:655112 & CN 100 355 413 C (MEICHEN GROUP CO LTD [CN]) 19 décembre 2007 (2007-12-19)

## Description

La présente invention concerne l'utilisation de composés 4-(3-éthoxy-4-hydroxyphényl)alkylcétone ou 2-éthoxy 4-hydroxyalkylphénol, susceptibles d'être dérivés de vanilline, comme agent antipelliculaire, en particulier dans le traitement cosmétique des états pelliculaires liés à la prolifération excessive des levures du genre Malassezia sur le cuir chevelu. L'invention concerne également un procédé de traitement cosmétique destiné à éliminer et/ou réduire les pellicules, notamment celles provoquées par les levures du genre Malassezia, employant lesdits composés.

Les problèmes de pellicules touchent jusqu'à 50% de la population mondiale. Ils touchent aussi bien les hommes que les femmes et sont perçus comme ayant un impact psychosocial très négatif. L'apparition de pellicules est gênante tant du point de vue esthétique que des désagréments qu'elle provoque (démangeaisons, rougeurs, etc.), si bien que beaucoup de gens confrontés à ce problème à des degrés variables souhaitent s'en débarrasser de manière efficace et définitive. Les pellicules correspondent à une desquamation excessive et visible du cuir chevelu, résultant de la multiplication trop rapide des cellules épidermiques et de leur maturation anormale. Ce phénomène peut être provoqué notamment par des microtraumatismes de nature physique ou chimique, tels que des traitements capillaires trop agressifs, des conditions climatiques extrêmes, la nervosité, l'alimentation, la fatigue, la pollution; mais il a été démontré que les états pelliculaires résultent le plus souvent d'un désordre de la microflore du cuir chevelu, et plus particulièrement de la colonisation excessive d'un champignon qui appartient à la famille des levures du genre Malassezia (autrefois appelées Pytirosporum ovale) et qui est naturellement présent sur le cuir chevelu.

De nombreux traitements antipelliculaires ont été développés avec pour principal objectif d'éradiquer les levures Malassezia du cuir chevelu. CN1513436 divulgue des shampooings contenant une huile résiné de gingembre comme agents antipelliculaires. Ainsi, l'activité des grands actifs antipelliculaires d'aujourd'hui, tels que le zinc pyrithione, la piroctone olamine ou le disulfure de sélénium, repose principalement sur leur propriété fongicide. Toutefois, ces agents antipelliculaires ne sont pas totalement satisfaisants en terme d'efficacité (efficacité immédiate ou durée de l'effet) et/ou en terme d'impact sur l'environnement.

La présente invention a pour but de proposer des agents antipelliculaires non irritants pour la peau et le cuir chevelu, aussi efficaces que les agents antipelliculaires connus, tout en ayant un impact plus favorable en terme d'environnement (faible bioaccumulation et bonne biodégradabilité notamment) et/ou présentant une bonne stabilité dans le temps (notamment après stockage d'une composition cosmétique le contenant, après 2 mois à température ambiante ou à 45 °C) et/ou présentant de bonnes propriétés cosmétiques telles qu'une bonne tolérance cutanée.

L'invention a également pour but de proposer des actifs permettant de rétablir l'écoflore du cuir chevelu et notamment de prévenir la colonisation excessive du cuir chevelu par Malassezia sp.

La demanderesse a maintenant trouvé de manière surprenante que l'utilisation d'au moins un composé de formule (I) permettait de traiter efficacement les états pelliculaires, notamment ceux associés à la prolifération de levures du genre Malassezia, et de remédier aux inconvénients de l'art antérieur.

Il a été observé qu'en employant les composés de formule (I), on pouvait éliminer et/ou réduire le nombre de levure du genre Malassezia, le nombre de pellicules, ainsi que les démangeaisons et les rougeurs sur le cuir chevelu.

La présente invention a donc pour objet l'utilisation, comme agent antipelliculaire, d'au moins un composé de formule (I) : 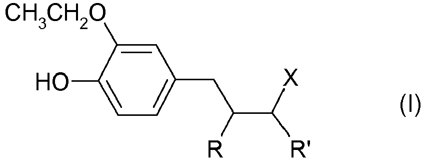 dans laquelle :
- R représente un atome d'hydrogène, ou un radical hydrocarboné, saturé ou insaturé (alkyle ou alcényle), linéaire ou ramifié, en C1-C6;
- R' représente un radical hydrocarboné, saturé ou insaturé (alkyle ou alcényle), linéaire ou ramifié, en C1-C18, éventuellement substitué par un groupe hydroxyle;
- C-X représente C=O ou CH-OH.

Notamment, l'invention concerne l'utilisation de composés de formule (I) pour traiter les états pelliculaires associés à la prolifération de levures du genre Malassezia sur le cuir chevelu.

De préférence, R représente H, méthyle ou éthyle.

De préférence, R' représente un radical hydrocarboné linéaire, saturé en C1-C6 ou insaturé en C2-C6, éventuellement substitué par un groupe hydroxyle.

De préférence, les composés répondent à la formule (I), dans laquelle :
- C-X représente C=O, R=H et R' représente un radical alkyle linéaire en C1-C6, éventuellement substitué par un OH; de préférence R'= méthyle ou éthyle; ou bien
- C-X représente CH-OH, R=H et R' représente un radical alkyle linéaire en C1-C6, éventuellement substitué par un OH; de préférence R'= méthyle ou éthyle.

On peut particulièrement citer les composés suivants :

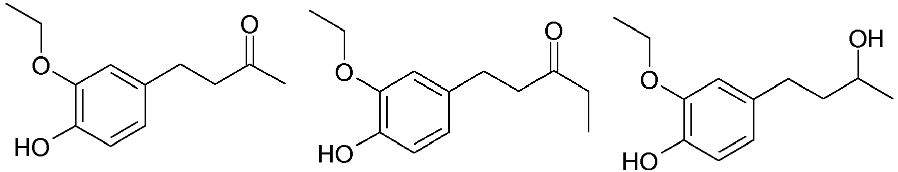

On peut bien évidemment utiliser un mélange de composés de formule (I).

Le composé particulièrement préféré est :

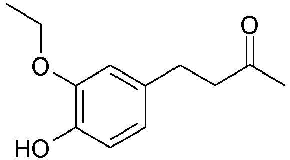

Les composés de formule (I) peuvent être préparés aisément par l'homme du métier sur la base de ses connaissances générales. On peut notamment citer les références bibliographiques suivantes : J. Asian Natural Products Research, 2006, 8(8), 683-688; Helv. Chimica Acta, 2006, 89(3), 483-495; Chem. Pharm. Bull., 2006, 54(3), 377-379; et Bioorg. Med. Chem. Lett., 2004, 14(5), 1287-1289.

Ils peuvent ainsi être préparés à partir d'éthylvanilline de la manière suivante :

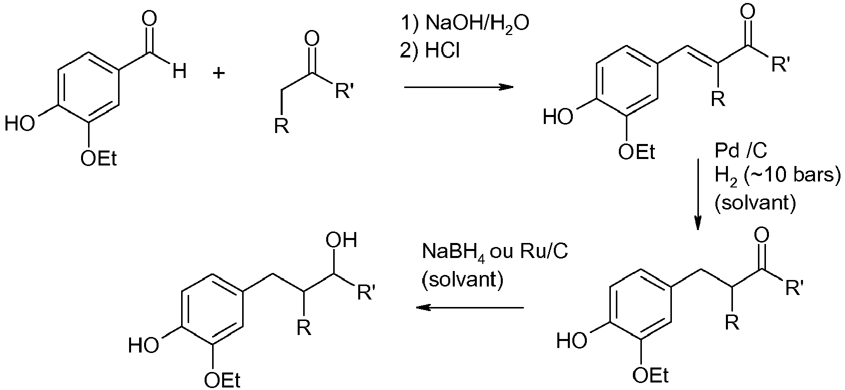

Les composés de formule (I) avec C-X représentant CHOH peuvent être obtenus par réduction des composés correspondants dans lesquels C-X représente C=O, par exemple par réduction avec Ru/C ou NaBH₄.

Les composés de formule (I), seul ou en mélange, peuvent être employés à raison de 0,1 à 10% en poids, notamment 0,5 à 5% en poids, par rapport au poids de la composition cosmétique.

Les compositions cosmétiques comprennent un composé de formule (I) et un milieu cosmétiquement acceptable, c'est-à-dire un milieu compatible avec les matières kératiniques telles que la peau du visage ou du corps, les lèvres, les cheveux, les cils, les sourcils et les ongles.

Les composés de formule (I) sont généralement utilisés en application topique.

En particulier, ils peuvent être utilisés comme agents antipelliculaires dans une composition cosmétique pouvant se présenter sous toutes les formes galéniques normalement utilisées pour une application topique.

La composition cosmétique utilisée peut être une composition capillaire, qui peut être rincée ou non rincée. Ladite composition capillaire est de préférence un shampooing, une crème, une mousse (aérosol ou non), une pâte, un gel, une émulsion, une lotion, un stick. De préférence, la composition cosmétique est un shampooing ou un gel.

La composition cosmétique utilisée comprend en général un milieu cosmétiquement acceptable. De préférence, ledit milieu comprend de l'eau et/ou un ou plusieurs solvants organiques cosmétiquement acceptables. Les solvants organiques peuvent être choisis parmi les monoalcools, linéaires ou ramifiés, en C1-C6 tels que l'éthanol, l'isopropanol, le tertio-butanol ou le n-butanol; les polyols tels que le glycérol, le propylèneglycol, l'hexylène glycol (ou 2-méthyl-2,4-pentanediol), et les polyéthylèneglycols; les éthers de polyols comme le monomé-thyléther de dipropylèneglycol; et leurs mélanges.

De préférence, la composition cosmétique utilisée comprend une quantité de solvants organiques allant de 0,05% à 60%, de préférence de 0,5 à 50%, et mieux encore de 1 à 40% en poids, par rapport au poids total de la composition cosmétique.

Le milieu cosmétiquement acceptable peut en outre avantageusement comprendre des épaississants; des tensioactifs choisis parmi les agents tensioactifs anioniques, cationiques, non ioniques et/ou amphotères ou zwittérioniques; des agents de conditionnement; des silicones; des agents antichute; des agents antipelliculaires autres; des agents oxydants, des vitamines; des cires, des filtres solaires, des pigments minéraux ou organiques, colorés ou non colorés; des colorants; des agents nacrants et opacifiants, des agents séquestrants, des agents plastifiants, des parfums; des conservateurs. Bien entendu, l'homme du métier veillera à choisir ce ou ces éventuels composés complémentaires et/ou leur quantité de manière telle que les propriétés avantageuses de la composition ne soient pas, ou substantiellement pas, altérées par l'adjonction envisagée.

L'invention a encore pour objet un procédé de traitement cosmétique destiné à éliminer et/ou réduire les pellicules, notamment celles provoquées par les levures du genre Malassezia, caractérisé en ce qu'il comprend l'application, sur les cheveux et/ou le cuir chevelu, d'un moins un composé de formule (I) ou bien d'une composition cosmétique comprenant au moins un composé de formule (I). La composition cosmétique peut ensuite être rincée ou non à l'eau. De préférence, on répète ce procédé de traitement cosmétique à raison d'au moins deux fois par semaine.

L'invention est illustrée plus en détail dans les exemples suivants.

### Exemple 1

### Composé testé :

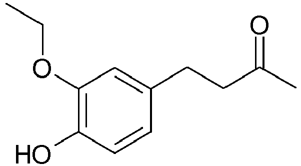

On prépare une solution à 2% en poids de composé à tester dans du "Leeming et Notman modifié liquide (MLNA)" de la manière suivante: on pèse 0,2048 g de composé à tester dans qsp 10 ml de Leeming et Notman modifié liquide; on solubilise par chauffage et utilisation des Ultrasons.

Les solutions de produit à tester sont deux fois plus concentrées que la concentration finale de test, afin de tenir compte de la dilution lors de la mise en contact avec la suspension de Malassezia.

L'une des solutions à 2% préalablement préparées est diluée au 1/2 dans du Leeming et Notman modifié liquide, pour obtenir au final une concentration de test de 0,5%; l'autre solution est employée telle quelle (à 2%) pour obtenir au final une concentration de test de 1%.

On met en contact les souches Malassezia suivant le tableau ci dessous :

| | Témoin de croissance | Composition à tester |
|---|---|---|
| Souche | 0,5 ml | 0,5 ml |
| Solution à tester | -- | 0,5 ml |
| Milieu MLNA | 0,5 ml | -- |

On agite et on dépose à la surface de la gélose MLNA, le mélange. On étale avec un râteau stérile sur toute la surface avant de récupérer l'excédent. On laisse incuber au moins 5 jours à 30°C.

L'effet antifongique du composé est évalué par l'absence de croissance de la souche Malassezia testée. Cette inhibition est évaluée par rapport au témoin de croissance. Les inhibitions sont notées de 0 à 3, par appréciation de la densité de la culture à la surface de la gélose en comparaison avec le témoin de croissance, de la manière suivante :

| Notation | Inhibition | Interprétation |
|---|---|---|
| 3 | 100% | Pas de croissance |
| 2 | 75% | Croissance < à la boîte témoin |
| 1 | 25% | Croissance < à la boîte témoin |
| 0 | 0% | Croissance comparable à la boîte témoin |

On obtient les résultats suivants :

| | Malassezia restricta | Malassezia globosa |
|---|---|---|
| Témoin de croissance | Culture dense | Culture dense |
| Composé testé à 1 % | 3 | 3 |
| Composé testé à 0,5% | 3 | 1 |

Le composé à 1% inhibe totalement la croissance des deux souches de Malassezia. Il est également très efficace à 0,5% sur Malassezia restricta et faiblement sur Malassezia globosa.

## Revendications

1. Utilisation, comme agent antipelliculaire, d'au moins un composé de formule (I) : 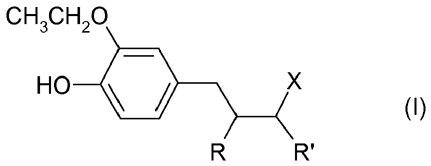 dans laquelle :
- R représente un atome d'hydrogène, ou un radical hydrocarboné, saturé ou insaturé, linéaire ou ramifié, en C1-C6;
- R' représente un radical hydrocarboné, saturé ou insaturé, linéaire ou ramifié, en C1-C18, éventuellement substitué par un groupe hydroxyle;
- C-X représente C=O ou CH-OH.

2. Utilisation selon la revendication 1, dans laquelle R représente H, méthyle ou éthyle.

3. Utilisation selon l'une des revendications précédentes, dans laquelle R' représente un radical hydrocarboné linéaire, saturé en C1-C6 ou insaturé en C2-C6, éventuellement substitué par un groupe hydroxyle.

4. Utilisation selon l'une des revendications précédentes, dans laquelle les composés répondent à la formule (I), dans laquelle :
- C-X représente C=O, R=H et R' représente un radical alkyle linéaire en C1-C6, éventuellement substitué par un OH; de préférence R'= méthyle ou éthyle; ou bien
- C-X représente CH-OH, R=H et R' représente un radical alkyle linéaire en C1-C6, éventuellement substitué par un OH; de préférence R'= méthyle ou éthyle.

5. Utilisation selon l'une des revendications précédentes, dans laquelle les composés de formule (I) sont choisis parmi les composés suivants :

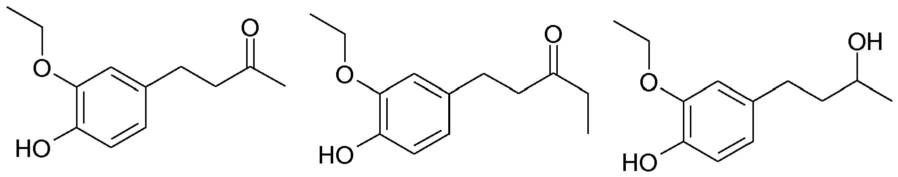

6. Utilisation selon l'une des revendications précédentes, dans laquelle les composés de formule (I) est :

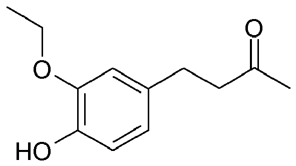

7. Utilisation selon l'une des revendications précédentes, dans laquelle le composé de formule (I), seul ou en mélange, est présent à raison de 0,1 à 10% en poids, notamment 0,5 à 5% en poids, par rapport au poids de la composition cosmétique.

8. Utilisation selon l'une des revendications précédentes, dans laquelle le composé de formule (I), seul ou en mélange, est présent dans une composition cosmétique capillaire comprenant un milieu cosmétiquement acceptable.

9. Utilisation selon l'une des revendications précédentes, dans laquelle le composé de formule (I), seul ou en mélange, est présent dans une composition cosmétique comprenant un milieu cosmétiquement acceptable qui comprend au moins un ingrédient choisi parmi : l'eau, les monoalcools, linéaires ou ramifiés, en C1-C6; les polyols; les éthers de polyols; des épaississants; des tensioactifs choisis parmi les agents tensioactifs anioniques, cationiques, non ioniques et/ou amphotères ou zwittérioniques; des agents de conditionnement; des silicones; des agents antichute; des agents antipelliculaires autres; des agents oxydants, des vitamines; des cires, des filtres solaires, des pigments minéraux ou organiques, colorés ou non colorés; des colorants; des agents nacrants et opacifiants, des agents séquestrants, des agents plastifiants, des parfums; des conservateurs.

10. Utilisation selon l'une des revendications précédentes, pour traiter les états pelliculaires associés à la prolifération de levures du genre Malassezia sur le cuir chevelu.

11. Procédé de traitement cosmétique destiné à éliminer et/ou réduire les pellicules, notamment celles provoquées par les levures du genre Malassezia, **caractérisé en ce qu'**il comprend l'application, sur les cheveux et/ou le cuir chevelu, d'au moins un composé de formule (I) tel que défini à l'une des revendications précédentes.

## Patentansprüche

1. Verwendung mindestens einer Verbindung der Formel (I) : 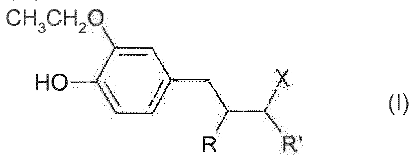 in der:
- R für ein Wasserstoffatom oder einen linearen oder verzweigten, gesättigten oder ungesättigten C₁-C₆-Kohlenwasserstoffrest steht;
- R' für einen linearen oder verzweigten, gesättigten oder ungesättigten C₁-C₁₈-Kohlenwasserstoffrest, der gegebenenfalls durch eine Hydroxylgruppe substituiert ist, steht;
- C-X für C=O oder CH-OH steht;
als Antischuppenmittel.

2. Verwendung nach Anspruch 1, wobei R für H, Methyl oder Ethyl steht.

3. Verwendung nach einem der vorhergehenden Ansprüche, wobei R' für einen linearen gesättigten C₁-C₆-Kohlenwasserstoffrest oder ungesättigten C₂-C₆-Kohlenwasserstoffrest, der gegebenenfalls durch eine Hydroxylgruppe substituiert ist, steht.

4. Verwendung nach einem der vorhergehenden Ansprüche, wobei die Verbindungen der Formel (I) entsprechen, in der:
- C-X für C=O steht, R = H und R' für einen linearen C₁-C₆-Alkylrest, der gegebenenfalls durch ein OH substituiert ist, steht; vorzugsweise R' = Methyl oder Ethyl; oder auch
- C-X für CH-OH steht, R = H und R' für einen linearen C₁-C₆-Alkylrest, der gegebenenfalls durch ein OH substituiert ist, steht; vorzugsweise R' = Methyl oder Ethyl.

5. Verwendung nach einem der vorhergehenden Ansprüche, wobei die Verbindungen der Formel (I) aus den folgenden Verbindungen ausgewählt sind:

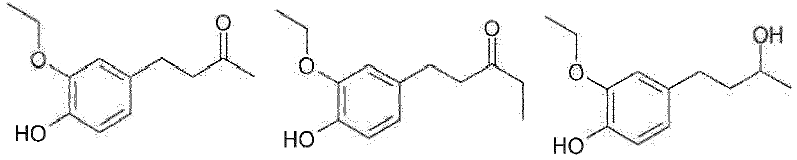

6. Verwendung nach einem der vorhergehenden Ansprüche, wobei es sich bei der Verbindung der Formel (I) um: 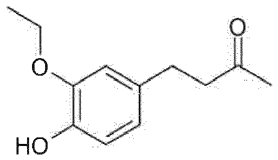 handelt.

7. Verwendung nach einem der vorhergehenden Ansprüche, wobei die Verbindung der Formel (I), alleine oder als Gemisch, in einer Menge von 0,1 bis 10 Gew.-%, insbesondere 0,5 bis 5 Gew.-%, bezogen auf das Gewicht der kosmetischen Zusammensetzung, vorliegt.

8. Verwendung nach einem der vorhergehenden Ansprüche, wobei die Verbindung der Formel (I), alleine oder als Gemisch, in einer haarkosmetischen Zusammensetzung, die ein kosmetisch unbedenkliches Medium umfasst, vorliegt.

9. Verwendung nach einem der vorhergehenden Ansprüche, wobei die Verbindung der Formel (I), alleine oder als Gemisch, in einer kosmetischen Zusammensetzung, die ein kosmetisch unbedenkliches Medium umfasst, vorliegt, welche mindestens einen Bestandteil umfasst, der aus Wasser, linearen oder verzweigten C₁-C₆-Monoalkoholen; Polyolen; Polyolethern; Verdickern; Tensiden, die aus anionischen, kationischen, nichtionischen und/oder amphoteren oder zwitterionischen Tensiden ausgewählt sind; Konditionierungsmitteln; Silikonen; Mitteln zur Bekämpfung von Haarverlust; anderen Antischuppenmitteln; Oxidationsmitteln; Vitaminen; Wachsen, Lichtschutzmitteln, farbigen oder farblosen anorganischen und organischen Pigmenten; Farbmitteln; Perlglanz- und Trübungsmitteln, Sequestriermitteln, Weichmachern, Parfümen und Konservierungsmitteln ausgewählt ist.

10. Verwendung nach einem der vorhergehenden Ansprüche zur Behandlung von Schuppenleiden, die mit der Proliferation von Hefen des Genus Malassezia auf der Kopfhaut assoziiert sind.

11. Kosmetisches Behandlungsverfahren zur Beseitigung und/oder Verringerung von Schuppen, insbesondere denjenigen, die durch Hefen des Genus Malassezia verursacht werden, **dadurch gekennzeichnet, dass** man mindestens eine Verbindung der Formel (I) gemäß einem der vorhergehenden Ansprüche auf die Haare und/oder die Kopfhaut aufbringt.

## Claims

1. Use, as an antidandruff agent, of at least one compound of formula (I): 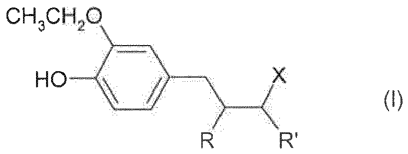 in which:
- R represents a hydrogen atom, or a linear or branched, saturated or unsaturated, C₁-C₆ hydrocarbon-based radical;
- R' represents a linear or branched, saturated or unsaturated, C₁-C₁₈ hydrocarbon-based radical, optionally substituted with a hydroxyl group;
- C-X represents C=O or CH-OH.

2. Use according to Claim 1, in which R represents H, methyl or ethyl.

3. Use according to either of the preceding claims, in which R' represents a saturated C₁-C₆, or unsaturated C₂-C₆, linear hydrocarbon-based radical, optionally substituted with a hydroxyl group.

4. Use according to one of the preceding claims, in which the compounds correspond to formula (I), in which:
- C-X represents C=O, R=H and R' represents a linear C₁-C₆ alkyl radical, optionally substituted with an OH; preferably, R' = methyl or ethyl; or else
- C-X represents CH-OH, R=H and R' represents a linear C₁-C₆ alkyl radical, optionally substituted with an OH; preferably, R' = methyl or ethyl.

5. Use according to one of the preceding claims, in which the compounds of formula (I) are chosen from the following compounds:

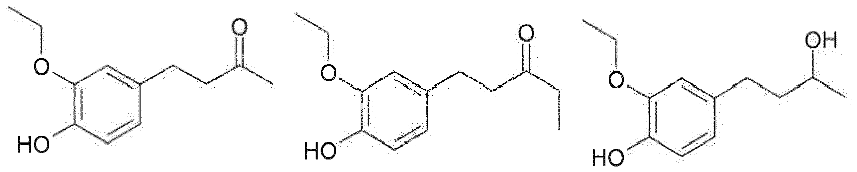

6. Use according to one of the preceding claims, in which the compound of formula (I) is:

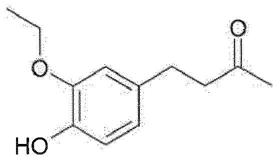

7. Use according to one of the preceding claims, in which the compound of formula (I), alone or as a mixture, is present in a proportion of from 0.1% to 10% by weight, in particular 0.5% to 5% by weight, relative to the weight of the cosmetic composition.

8. Use according to one of the preceding claims, in which the compound of formula (I), alone or as a mixture, is present in a cosmetic hair composition comprising a cosmetically acceptable medium.

9. Use according to one of the preceding claims, in which the compound of formula (I), alone or as a mixture, is present in a cosmetic composition comprising a cosmetically acceptable medium which comprises at least one ingredient chosen from: water, linear or branched C₁-C₆ monoalcohols; polyols; polyol ethers; thickeners; surfactants chosen from anionic, cationic, non-ionic and/or amphoteric or zwitterionic surfactants; conditioning agents; silicones; agents for combating hair loss; other antidandruff agents; oxidizing agents, vitamins; waxes; sunscreens; coloured or colourless inorganic or organic pigments; dyes; pearlescent and opacifying agents; sequestering agents; plasticizers; fragrances; preservatives.

10. Use according to one of the preceding claims, for treating the dandruff conditions associated with the proliferation of yeasts of the Malassezia genus on the scalp.

11. Cosmetic treatment method intended to eliminate and/or reduce dandruff, in particular dandruff caused by yeasts of the Malassezia genus, **characterized in that** it comprises the application, to the hair and/or the scalp, of at least one compound of formula (I) as defined in one of the preceding claims.
